# NOUVEAU FASCICULE DE BREVET EUROPEEN

(11) **EP 0 663 203 B2**
(45) Date de publication et mention de la décision concernant l'opposition: **17.03.2010**
(45) Mention de la délivrance du brevet: 19.09.2001
(21) Numéro de dépôt: 94402874.5
(22) Date de dépôt: 13.12.1994
(51) Int. Cl.: A61Q 5/06, A61K 8/02, A61K 8/72

(54) **Compositions cosmétiques en aérosol, et utilisations**
Kosmetische Zusammensetzungen, die in einem Aerosolbehälter verpackt sind, und ihre Verwendungen
Cosmetic aerosol compositions and its use

(30) Priorité: 14.01.1994 FR 9400367
(43) Date de publication de la demande: 19.07.1995
(73) Titulaire: L'OREAL, S.A., 75008 Paris (FR)
(72) Inventeur: Le Bourhis, François, F-93300 Aubervilliers (FR)
(74) Mandataire: Dossmann, Gérard

(56) Documents cités:
- EP-A- 0 251 462
- EP-A- 0 257 444
- EP-A- 0 455 081
- EP-A- 0 480 280
- EP-A- 0 523 388
- EP-A- 0 557 087
- EP-A- 0 574 607
- DE-A- 3 524 263
- DE-A- 4 034 315
- GB-A- 2 098 226
- JP-A- 04 112 819
- K. SCHRADER: 'Grundlagen und Rezepturen der Kosmetika', 1989, HÜTHIG BUCH VERLAG, HEIDELBERG page 54,108
- AEROSOL REPORT, Vol. 30, No. 5/91, H. Berkhout "Haarspray-Formulierungen mit Dimethylether", pp. 258-281
- SEIFEN-ÖLE-FETTE-WACHSE, 117 Jg. Nr. 13/1991, Guth et al. "Addressing the North American Trend Toward Low VOC Hair Sprays", pp.464-467
- RESEARCH DISCLOSURE, May 1985, Nr. 25349, Ciba-Geigy AG, Basle

## Description

La présente invention est relative à de nouvelles compositions cosmétiques pressurisées en aérosol et formant spontanément, au contact d'une surface, en particulier les cheveux, une mousse.

Différentes formes de présentation sont utilisées pour dispenser les produits cosmétiques sur les matieres kératiniques à traiter. En particulier, les produits conditionnés en aérosol et produisant instantanément de la mousse à la sortie de la tête de distribution du récipient les contenant, sont largement utilisées dans le domaine des produits capillaires tels que les produits de coiffage ou de soin.

Le mode d'utilisation de ces produits pressurisés en aérosol consiste à prélever de la mousse dans la main, à sa sortie du récipient, et à appliquer cette mousse sur les cheveux en la répartissant avec les mains.

Toute manipulation supplémentaire du récipient, par exemple pour ajouter de la mousse dans la main, entraîne une salissure de celui-ci et/ou un risque de le faire tomber. Il est donc nécessaire de se laver les mains après usage et ceci encore plus particulièrement si l'on doit utiliser un sèche-cheveux pour sécher les cheveux et/ou faire un brushing.

La demanderesse a maintenant découvert qu'une solution à ce problème consiste à ne plus mettre la mousse directement sur la main, mais à pulvériser une composition particulière qui sort du récipient sous la forme d'un nuage de particules liquides et se transforme ensuite spontanément, mais avec un certain temps de retard à compter de la sortie du récipient, en mousse au contact de la surface d'application.

L'invention concerne donc une composition cosmétique pressurisée en aérosol et formant spontanément une mousse au contact de la surface sur laquelle elle est appliquée, en particulier les cheveux, caractérisée par le fait qu'elle comprend (i) un support liquide aqueux contenant au moins un polymère anionique à pouvoir moussant et/ou un polymère cationique à pouvoir moussant, et (ii) un mélange de propulseurs contenant du diméthyléther en association avec au moins un autre propulseur choisi parmi les alcanes volatiles, les halogénoalcanes volatiles et leurs mélanges; le solvent étant présent dans une quantité inférieure à 20 % en poids par rapport au poids total de la composition et le rapport en poids diméthylétherlautre(s) propulseur(s) variant de 95/5 à 50/50, la composition contenant 20 à 90% en poids d'eau par rapport au poids total de la composition, le dimethyléther étant présent dans des quantités allant de 4 à 35% en poids par rapport au poids total de la composition.

Selon l'invention, la mousse ne se forme pas à la sortie du récipient mais seulement au contact de la surface d'application (effet retard). Il est donc facile de localiser la mousse aux endroits souhaités. La pulvérisation d'un nuage de particules permet en outre une répartition homogène. En l'absence de diméthyléther, l'effet retard mentionné ci-dessus ne peut être obtenu.

Selon l'invention, les compositions peuvent en outre contenir des ingrédients habituellement mis en oeuvre dans les compositions cosmétiques, mais il est nécessaire que ces derniers n'empêchent pas la formation de la mousse.

Cette mousse est de préférence éphémère, c'est à dire qu'elle disparaît rapidement après sa formation, sans qu'il soit nécessaire de la travailler avec les mains.

L'invention concerne également un procédé de traitement des matières kératiniques, en particulier des cheveux, à l'aide de cette composition.

Elle concerne aussi un récipient aérosol contenant une composition telle que définie ci-dessus.

On entend par polymère à pouvoir moussant conforme à l'invention, un polymère qui en solution dans l'eau à 0,5% en poids donne selon le test de Ross Miles (norme AFNOR T 73 404) modifié en température et effectué à 20°C, une hauteur de mousse supérieure à 1 cm et après pressurisation de la solution une quantité de mousse telle que sa masse volumique soit inférieure à 0,4 et de préférence inférieure à 0,25 g/cm³. Ces tests sont décrits dans le brevet français 2505348 qui est inclus à titre de référence.

Selon l'invention, on peut utiliser tout polymère anionique ou cationique connu en soi répondant à ces tests.

Ainsi, les polymères anioniques généralement utilisés sont des polymères comportant des groupements dérivés d'acide carboxylique, sulfonique ou phosphorique et ont un poids moléculaire compris entre environ 500 et 5.000.000.

Les groupements carboxyliques sont apportés par des monomères mono ou diacides carboxyliques insaturés tels que ceux répondant à la formule : 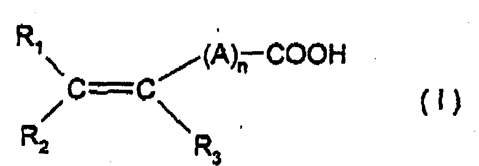 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂ - COOH, phényle ou benzyle.

Dans la formule précitée un radical alkyle inférieur désigne de préférence un groupement ayant 1 à 4 atomes de carbone et en particulier, méthyle et éthyle.

Les polymères anioniques moussants à groupements carboxyliques préférés selon l'invention sont :
A) les homo- ou copolymères d'acide acrylique ou méthacrylique ou leurs sels et en particulier les produits vendus sous les dénominations VERSICOL E ou K par la société ALLIED COLLOID, ULTRAHOLD par la société BASF, les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀.par exemple de lauryle tels que celui vendus par la société ISP sous la dénomination ACRYLIDONE LM.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées. Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, lignosulfonique, naphtalène sulfonique ou acrylamido alkylsulfonique.

Ces polymères peuvent être notamment choisis parmi :
- les sels de l'acide polyvinylsufonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels de métaux alcalins et alcalino terreux des acides sulfoniques dérivés de la lignine, et en particulier les lignosulfonates de calcium ou de sodium tels que le produit vendu sous la dénomination Marasperse C-21 par American Can Co. et ceux en C₁₀-C₁₄ vendus par Avébène.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères anioniques sont de préférence choisis parmi les copolymères d'acide acrylique tels que le terpolymère acide acryliquelacrylate d'éthyle/N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que la copolymère méthylvinyléther/anhydride maléique mono estérifié vendu sous la dénomination GANTREZ ES 425 par la société ISP.

Selon l'invention, les polymères cationiques sont par exemple décrits dans le brevet français 2505348 qui est inclus à titre de référence.

Les polymères cationiques à pouvoir moussant sont de préférence choisis parmi les composés suivants :
- les copolymères polyamine/polyglycol réticulés tels que les produits vendus sous les dénominations POLYQUART par la société HENKEL;
- les éthers de cellulose cationiques répondant à la formule (II) suivante : 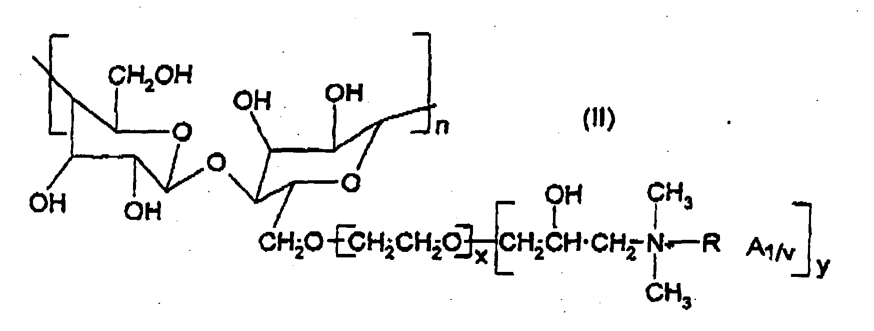 dans laquelle R est un radical alkyle ayant au moins 8 atomes de carbone, de préférence 10 à 24 atomes de carbone et mieux 10 à 18 atomes de carbone, A⁻ est un anion ou un mélange d'anions, v est égal à la valence de A, n est un nombre entier compris entre 50 et 20000, de préférence 100 à 6000 et mieux encore 250 à 4000, x est un nombre entier allant de 0 à 6, et y est un nombre entier allant de 0 à 3, de préférence de 0 à 2, à la condition que le taux de substitution cationique, CS soit supérieur à 0.
   Ces composés sont décrits ainsi que leur préparation dans la demande de brevet EP-A-189.935.
   Le taux de substitution cationique ( ou de quaternisation), CS, est défini comme la moyenne molaire des atomes d'azote quaternaire par motif récurrent de cellulose. Ce taux est supérieur à 0, de préférence inférieur à 1 et mieux compris entre 0,01 et 0,6.
   Un éther de cellulose cationique de formule (II) particulièrement préféré est vendu sous la dénomination QUATRISOFT LM 200 par la société AMERCHOL.

Selon un mode de réalisation préféré de l'invention, la composition contient un polymère anionique à pouvoir moussant, et encore plus particulièrement un polymère anionique à pouvoir moussant en association avec un polymère cationique moussant ou non moussant.

Une association polymère anionique/polymère cationique plus particulièrement préférée selon l'invention est une association comprenant un copolymère méthylvinyléther/anhydride maléique monoestérifié et un copolymère hydroxyéthylcelluloselchlorure de diallyl diméthyl ammonium.

Le ou les polymères sont par exemple présents dans des concentrations comprises entre 0,1% et 15% en poids, et de préférence dans des concentrations comprises entre 0,5% et 10% en poids, par rapport au poids total de la composition.

Selon l'invention, on entend par propulseur tout fluide qui bout à une température inférieure ou égale à 40,6°C.

Selon l'invention, l'alcane est de préférence choisi parmi l'isobutane, le propane, le butane, le pentane et l'isopentane, et l'halogénoalcane est de préférence choisi parmi les difluoroéthanes, les tétrafluoroéthanes et l'octafluorocyclobutane, et leurs mélanges.

Selon un mode de réalisation particulièrement préféré de la présente invention, le mélange de propulseurs est constitué par du diméthyléther et de l'isobutane.

Le mélange diméthyléther/autre(s) propulseur(s) est présent de préférence dans des quantités allant de 5 à 55% en poids par rapport au poids total de la composition et encore plus particulièrement dans des quantités allant de 15 à 35% en poids.

Le diméthyléther est présent de préférence dans des quantités allant de 4 à 35% en poids par rapport au poids total de la composition.

Le ou les autres propulseurs sont présents de préférence dans des quantités allant de 1 à 20% en poids par rapport au poids total de la composition, et de préférence de 2 à 15% en poids.

Le rapport en poids diméthyléther/ autre(s) propulseur(s) peut varier de 95/5 à 50/50 et de préférence de 90/10 à 55/45.

Le support liquide aqueux utilisé pour la préparation des compositions conformes à l'invention doit permettre la formation d'une mousse de préférence éphémère au contact des cheveux.

Selon l'invention, le support est par exemple constitué par de l'eau et peut contenir éventuellement des solvants cosmétiquement acceptables choisis notamment parmi l'éthanol et l'isopropanol.

La quantité de solvant est telle qu'elle ne perturbe pas le caractère moussant de la composition, cette quantité est inférieure à 20% en poids par rapport au poids total de la composition.

Selon l'invention, la quantité d'eau est de préférence comprise entre 20 et 90% et encore plus particulièrement entre 40 et 80% en poids par rapport au poids total de la composition.

Les compositions selon l'invention peuvent en outre contenir des ingrédients habituellement mis en oeuvre dans les compositions cosmétiques, pour autant que ces derniers ne perturbent pas le caractère moussant des compositions selon l'invention ; ce sont par exemple des peptisants, des agents tensioactifs, des agents traitants, des agents alcalinisants ou acidifiants, des agents conservateurs, des parfums, des silicones (volatiles ou non, fonctionnalisées ou non), des agents anticorrosion, des colorants, des filtres solaires, des protéines, des vitamines, d'autres polymères, des huiles végétales, animales, minérales ou synthétiques et tout autre additif utilisé dans ce type de composition.

Un des avantages des compositions selon l'invention est qu'elles peuvent être exemptes de tout tensioactif.

Les compositions de l'invention sont utilisables plus spécialement comme composition capillaire dont l'application sur cheveux secs ou humides est suivie éventuellement d'un rinçage à l'eau. Aussi, l'invention se rapporte à l'utilisation de cette composition comme composition à appliquer avant ou après tout traitement capillaire tel qu'un shampooing, une coloration ou une décoloration, une permanente ou un défrisage.

La présente invention se rapporte encore plus particulièrement à l'utilisation comme composition capillaire dont l'application n'est pas suivie d'un rinçage à l'eau, comme composition de mise en plis, de brushing, de mise en forme ou de coiffage.

L'invention se rapporte également à l'utilisation de cette composition comme composition de coloration, de décoloration, de permanente ou de défrisage des cheveux.

L'invention a encore pour objet un procédé de traitement des matières kératiniques consistant à appliquer sur lesdites matières une composition telle que définie précédemment.

Dans les exemples qui suivent, on donne des compositions concrètes conformes à l'invention.

### Exemple 1

On a préparé une composition présentant les caractéristiques suivantes :
- Copolymère méthylvinyléther/monomaléate de butyle en solution dans l'éthanol à 50% de matière active vendu sous la dénomination GANTREZ ES 425 par la société ISP 4 gMA
- Amino-2 méthyl-2 propanol qs pH 8,5
- Parfum, conservateur qs
- Eau qsp 100 g

Cette composition est conditionnée selon le schéma de pressurisation suivant:
- Composition ci-dessus : 70 %
- Isobutane 10 %
- Diméthyléther 20 %

La composition pressurisée est pulvérisée sur les cheveux humides sous la forme d'un nuage de particules liquides, une mousse se développe alors sur les cheveux. Cette mousse non poisseuse, disparaît rapidement sur les cheveux. Les cheveux séchés sont doux au toucher et ont une bonne tenue.

### Exemple 2

On a préparé une composition présentant les caractéristiques suivantes :
- Terpolymère acide acrylique/acrylate d'éthyle/N-tertiobutylacrylamide vendu sous la dénomination ULTRAHOLD STRONG par la société BASF 2 g
- Amino-2 méthyl-2 propanol qs pH 8
- Parfum, conservateur qs
- Eau qsp 100 g

Cette composition est conditionnée selon le schéma de pressurisation suivant:
- Composition ci-dessus : 75 %
- Isobutane 8 %
- Diméthyléther 17 %

Pulvérisée sur cheveux humides, cette composition donne les mêmes résultats qu'à l'exemple 1.

### Exemple 3

On a préparé une composition présentant les caractéristiques suivantes :
- Copolymère méthylvinyléther/monomaléate de butyle en solution dans l'éthanol à 50% de matière active vendu sous la dénomination GANTREZ ES 425 par la société ISP 3,5 gMA
- Copolymère hydroxyéthylcellulose/chlorure de diallyl diméthyl ammonium vendu sous la dénomination CELQUAT L 200 par la société NATIONAL STARCH 2g
- Amino-2 méthyl-2 propanol qs pH 8
- Ethanol 10 g
- Parfum, conservateur qs
- Eau qsp 100 g

Cette composition est conditionnée selon le schéma de pressurisation suivant:
- Composition ci-dessus : 70 %
- n-butane 10 %
- Diméthyléther 20 %

Pulvérisée sur cheveux humides, cette composition donne les mêmes résultats qu'à l'exemple 1.

### Exemple 4

On a préparé une composition présentant les caractéristiques suivantes :
- Hydroxyéthylcellulose quaternisée (Polyquatemium-24 selon la nomenclature CTFA 4ème éd. 1992) vendue sous la dénomination QUATRISOFT LM 200 par la société AMERCHOL 1 g
- Parfum, conservateur qs
- Eau qsp 100 g

Cette composition est conditionnée selon le schéma de pressurisation suivant:
- Composition ci-dessus : 75 %
- n-butane 10 %
- Diméthyléther 15 %

Pulvérisée sur cheveux humides, cette composition donne les mêmes résultats qu'à l'exemple 1.

## Revendications

1. Composition cosmétique pressurisée en aérosol formant spontanément une mousse au contact d'une surface d'application, en particulier les cheveux, **caractérisée par le fait qu'**elle comprend (i) un support aqueux contenant au moins un polymère anionique à pouvoir moussant et/ou un polymère cationique à pouvoir moussant, et (ii) un mélange de propulseurs contenant du diméthyléther en association avec au moins un autre propulseur choisi parmi les alcanes volatiles, les halogénoalcanes volatiles et leurs mélanges; le solvant étant présent dans une quantité inférieure à 20 % en poids par rapport au poids total de la composition et le rapport en poids diméthyléther/autre(s) propulseur(s) variant de 95/5 à 50/50, la composition contenant 20 à 90 % en poids d'eau par rapport au poids total de la composition, le diméthyléther étant présent dans des quantités allant de 4 à 35 % en poids par rapport au poids total de la composition.

2. Composition selon la revendication 1, **caractérisée en ce que** le polymère anionique est choisi parmi :
- les polymères comportant des motifs carboxyliques dérivant de monomères mono ou diacides carboxyliques insaturés de formule : 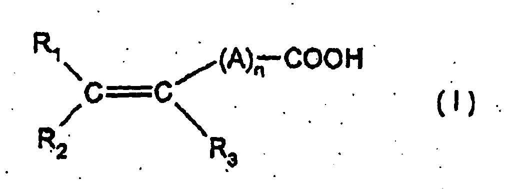 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂ - COOH, phényle ou benzyle.
- les polymères comprenant des motifs dérivant d'acide sulfonique tels que des motifs vinylsulfonique, styrènesulfonique, lignosulfonique, acrylamido alkylsulfonique.

3. Composition selon l'une quelconque des revendications précédentes, **caractérisée en ce que** le polymère anionique est choisi parmi :
A) les homo- ou copolymères d'acide acrylique ou méthacrylique ou leurs sels, les copolymères d'acide acrylique et d'acrylamide et leurs sels, les sels de sodium d'acides polyhydroxycarboxyliques.
B) Les copolymères des acides acrylique ou méthacryliques avec un monomère monoéthylénique tel que l'éthylène, le styrène, les esters vinyliques, les esters d'acide acrylique ou méthacrylique, éventuellement greffés sur un polyalcoylène glycol tel que le polyéthylène glycol et éventuellement réticulés ; les copolymères de ce type comportant dans leur chaîne un motif acrylamide éventuellement N-alkylé et/ou hydroxyalkylé, les copolymères d'acide acrylique et de méthacrylate d'alkyle en C₁-C₄ et les terpolymères de vinylpyrrolidone, d'acide acrylique et de méthacrylate d'alkyle en C₁-C₂₀.
C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés.
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées.
E) les polyacrylamides comportant des groupements carboxylates.

4. Composition selon l'une quelconque des revendications précédentes, **caractérisée en ce que** le polymère anionique est choisi parmi :
- les copolymères d'acide acrylique tels que le terpolymère acide acrylique/acrylate d'éthyle/N-tertiobutylacrylamide ;
- les copolymères dérivés d'acide crotonique tels que les terpolymères acétate de vinyle / tertio-butyl benzoate de vinyle / acide crotonique et les terpolymères acide crotoniquelacétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinylétherlanhydride maléique mono estérifié.

5. Composition selon l'une des revendications 1 à 4, **caractérisée en ce que** le polymère cationique moussant est choisi parmi :
- les copolymères polyamine/polyglycol réticulés,
- les éthers de cellulose cationiques répondant à la formule (II) suivante : 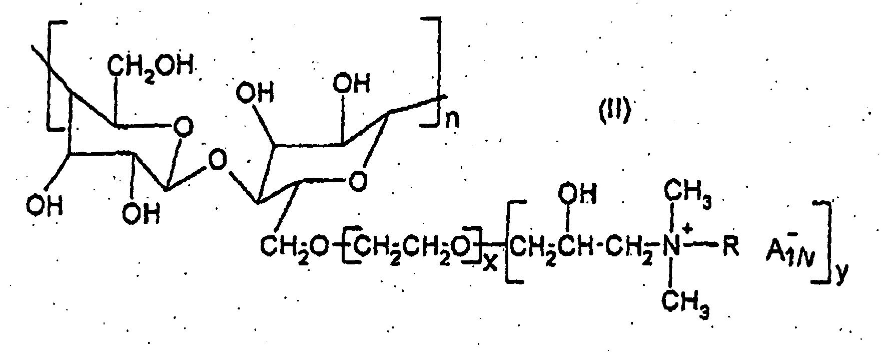 dans laquelle R est un radical alkyle ayant au moins 8 atomes de carbone, de préférence 10 à 24 atomes de carbone et mieux 10 à 18 atomes de carbone, A⁻ est un anion ou un mélange d'anions, v est égal à la valence de A, n est un nombre entier compris entre 50 et 20000, de préférence 100 à 6000 et mieux encore 250 à 4000, x est un nombre entier allant de 0 à 6, et y est un nombre entier allant de 0 à 3, de préférence de 0 à 2, à la condition que le taux de substitution cationique, CS soit suprérieur à 0.

6. Composition selon l'une quelconque des revendications précédentes, **caractérisée en ce que** le ou les polymères sont présents dans des concentrations comprises entre 0,1% et 15% en poids par rapport au poids total de la composition.

7. Composition selon la revendication 6, **caractérisée en ce que** le ou les polymères sont présents dans des concentrations comprises entre 0,5% et 10% en poids par rapport au poids total de la composition.

8. Composition selon l'une quelconque des revendications précédentes, **caractérisée en ce que** l'alcane est choisi parmi l'isobutane, le propane, le butane, le pentane, l'isopentane et leurs mélanges.

9. Composition selon l'une quelconque des revendications 1 à 7, **caractérisée en ce que** l'halogénoalcane est choisi parmi les difluoroéthanes, les tétrafluoroéthanes, l'octafluorocyclobutane et leurs mélanges.

10. Composition selon l'une quelconque des revendications 1 à 8, **caractérisée en ce que** le mélange de propulseurs est constitué par du diméthyléther et de l'isobutane.

11. Composition selon l'une quelconque des revendications précédentes, **caractérisée en ce que** le mélange diméthyléther/autre(s) propulseur(s) est présent dans des quantités allant de 5 à 55% en poids par rapport au poids total de la composition.

12. Composition selon la revendication 11, **caractérisée en ce que** le mélange diméthyléther/autre(s) propulseur(s) est présent dans des quantités allant de 15 à 35% en poids par rapport au poids total de la composition.

13. Composition selon l'une quelconque des revendications précédentes, **caractérisée en ce que** le ou les autres propulseurs sont présents dans des quantités allant de 1 à 20% en poids par rapport au poids total de la composition.

14. Composition selon l'une quelconque des revendications précédentes, **caractérisés en ce que** le rapport en poids diméthyléther/autre(s) propulseur(s): varie de 90/10 à 55/45.

15. Composition selon l'une quelconque des revendications précédentes, **caractérisée en ce qu'**elle comprend un polymère anionique à pouvoir moussant en association avec un polymère cationique moussant ou non.

16. Composition selon l'une quelconque des revendications précédentes, **caractérisée en ce qu'**elle comprend un copolymère méthylvinyléther/anhydride maléique monoestérifié et un copolymère hydroxyéthylcellulose/chlorure de diallyl diméthyl ammonium.

17. Récipient aérosol, **caractérisé en ce qu'**il comprend une composition telle que définie à l'une quelconque des revendications précédentes.

18. Procédé de traitement des matières kératiniques, **caractérisé par le fait que** l'on applique sur ces matières au moins une composition telle que définie à l'une quelconque des revendications 1 à 16.

19. Utilisation d'une composition selon l'une quelconque des revendications 1 à 16 comme composition à appliquer avant ou après tout traitement capillaire tel qu'un shampooing, une coloration ou une décoloration, une permanente ou un défrisage, ou comme composition de mise en plis, de brushing, de mise en forme ou de coiffage, ou comme composition de coloration, de décoloration, de permanente ou de défrisage des cheveux.

## Claims

1. Cosmetic composition pressurized as an aerosol spontaneously forming a foam on contact with an application surface, in particular the hair, **characterized in that** it comprises (i) an aqueous support containing at least one anionic polymer with foaming power and/or one cationic polymer with foaming power, and (ii) a mixture of propellants containing dimethyl ether in combination with at least one other propellant chosen from volatile alkanes and volatile haloalkanes and mixtures thereof, the solvent being present in an amount of less than 20% by weight relative to the total weight of the composition and the dimethyl ether/other propellant(s) weight ratio ranging from 95/5 to 50/50, the composition containing 20% to 90% by weight of water, relative to the total weight of the composition, the dimethyl ether being present in amounts ranging from 4% to 35% by weight relative to the total weight of the composition.

2. Composition according to Claim 1, **characterized in that** the anionic polymer is chosen from:
- polymers comprising carboxylic units derived from unsaturated monocarboxylic or dicarboxylic acid monomers of formula: 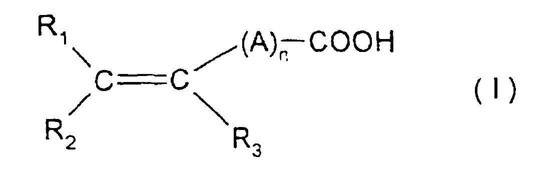 in which n is an integer from 0 to 10, A denotes a methylene group, optionally linked to the carbon atom of the unsaturated group or to the neighbouring methylene group when n is greater than 1 via a hetero atom such as oxygen or sulphur, R₁ denotes a hydrogen atom or a phenyl or benzyl group, R₂ denotes a hydrogen atom or a lower alkyl or carboxyl group, R₃ denotes a hydrogen atom, a lower alkyl group or a -CH₂-COOH, phenyl or benzyl group,
- polymers comprising units derived from sulphonic acid, such as vinylsulphonic, styrenesulphonic, lignosulphonic or acrylamidoalkylsulphonic units.

3. Composition according to either of the preceding claims, **characterized in that** the anionic polymer is chosen from:
A) homopolymers or copolymers of acrylic or methacrylic acid or salts thereof, copolymers of acrylic acid and of acrylamide and salts thereof, sodium salts of polyhydroxycarboxylic acids;
B) copolymers of acrylic or methacrylic acids with a monoethylenic monomer such as ethylene, styrene, vinyl esters, acrylic acid or methacrylic acid esters, optionally grafted onto a polyalkylene glycol such as polyethylene glycol and optionally crosslinked; the copolymers of this type comprising in their chain an optionally N-alkylated and/or hydroxyalkylated acrylamide unit, copolymers of acrylic acid and of a C₁-C₄ alkyl methacrylate and terpolymers of vinylpyrrolidone, of acrylic acid and of a C₁-C₂₀ alkyl methacrylate;
C) copolymers derived from crotonic acid, such as those comprising in their chain vinyl acetate or propionate units and optionally other monomers such as allylic or methallylic esters, vinyl ether or vinyl ester of a saturated, linear or branched carboxylic acid containing a long hydrocarbon-based chain, such as those comprising at least 5 carbon atoms, these polymers possibly being grafted and crosslinked;
D) polymers derived from maleic, fumaric or itaconic acids or anhydrides with vinyl esters, vinyl ethers, vinyl halides or phenylvinyl derivatives, and acrylic acid and its esters; copolymers of maleic, citraconic or itaconic anhydride and of an allylic or methallylic ester optionally comprising an acrylamide or methacrylamide group, an α-olefin, acrylic or methacrylic esters, acrylic or methacrylic acids or vinylpyrrolidone in their chain, the anhydride functions being monoesterified or monoamidated;
E) polyacrylamides comprising carboxylate groups.

4. Composition according to any one of the preceding claims, **characterized in that** the anionic polymer is chosen from:
- copolymers of acrylic acid, such as acrylic acid/ethyl acrylate/N-tert-butylacrylamide terpolymer;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acids or anhydrides with vinyl esters, vinyl ethers, vinyl halides or phenyl vinyl derivatives, and acrylic acid and its esters, such as methyl vinyl ether/monoesterified maleic anhydride copolymers.

5. Composition according to one of Claims 1 to 4, **characterized in that** the foaming cationic polymer is chosen from:
- crosslinked polyamine/polyglycol copolymers,
- cationic cellulose ethers corresponding to formula (II) below: 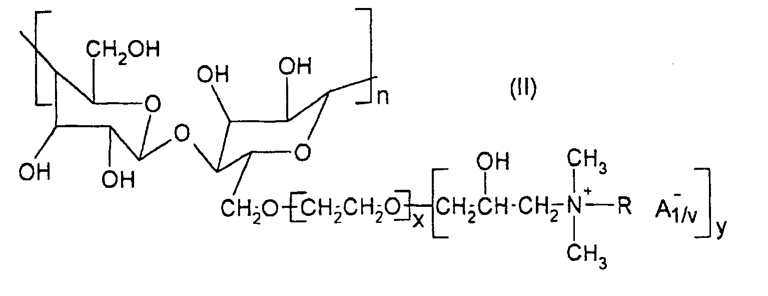 in which R is an alkyl radical containing at least 8 carbon atoms, preferably 10 to 24 carbon atoms and better still 10 to 18 carbon atoms, A⁻ is an anion or a mixture of anions, v is equal to the valency of A, n is an integer between 50 and 20 000, preferably from 100 to 6 000 and better still from 250 to 4 000, x is an integer ranging from 0 to 6 and y is an integer ranging from 0 to 3 and preferably from 0 to 2, on condition that the degree of cationic substitution, CS, is greater than 0.

6. Composition according to any one of the preceding claims, **characterized in that** the polymer(s) is(are) present in concentrations of between 0.1% and 15% by weight relative to the total weight of the composition.

7. Composition according to Claim 6, **characterized in that** the polymer(s) is (are) present in concentrations of between 0.5% and 10% by weight relative to the total weight of the composition.

8. Composition according to any one of the preceding claims, **characterized in that** the alkane is chosen from isobutane, propane, butane, pentane and isopentane, and mixtures thereof.

9. Composition according to any one of Claims 1 to 7, **characterized in that** the haloalkane is chosen from difluoroethanes, tetrafluoroethanes and octafluorocyclobutane, and mixtures thereof.

10. Composition according to any one of Claims 1 to 8, **characterized in that** the mixture of propellants consists of dimethyl ether and isobutane.

11. Composition according to any one of the preceding claims, **characterized in that** the dimethyl ether/other propellant(s) mixture is present in amounts of from 5% to 55% by weight relative to the total weight of the composition.

12. Composition according to Claim 12, **characterized in that** the dimethyl ether/other propellant(s) mixture is present in amounts ranging from 15% to 35% by weight relative to the total weight of the composition.

13. Composition according to any one of the preceding claims, **characterized in that** the other propellant(s) is(are) present in amounts ranging from 1% to 20% by weight relative to the total weight of the composition.

14. Composition according to any one of the preceding claims, **characterized in that** the dimethyl ether/other propellant(s) weight ratio ranges from 90/10 to 55/45.

15. Composition according to any one of the preceding claims, **characterized in that** it comprises an anionic polymer with foaming power in combination with a foaming or non-foaming cationic polymer.

16. Composition according to any one of the preceding claims, **characterized in that** it comprises a methyl vinyl ether/monoesterified maleic anhydride copolymer and a hydroxyethylcellulose/diallyldimethylammonium chloride copolymer.

17. Aerosol container, **characterized in that** it comprises a composition as defined in any one of the preceding claims.

18. Process for treating keratin materials, **characterized in that** at least one composition as defined in any one of Claims 1 to 16 is applied to these materials.

19. Use of a composition according to any one of Claims 1 to 16 as a composition to be applied before or after any hair treatment such as shampooing, dyeing, bleaching, permanent-waving or relaxing the hair, or as a hairsetting, blow-drying, shaping or styling composition, or as a dyeing, bleaching, permanent-waving or relaxing composition for the hair.

## Patentansprüche

1. Kosmetische Zusammensetzung, die in einem Aerosolbehälter unter Druck steht und die im Kontakt mit einer Anwendungsoberfläche, insbesondere den Haaren, spontan einen Schaum bildet, **dadurch gekennzeichnet, dass** sie umfaßt: (i) einen wäßrigen Träger, der mindestens ein anionisches Polymer mit Schaumvermögen und/oder mindestens ein kationisches Polymer mit Schaumvermögen enthält, und (ii) ein Gemisch von Treibmitteln, das Dimethylether in Kombination mit mindestens einem weiteren Treibmittel enthält, das unter flüchtigen Alkanen, flüchtigen Halogenalkanen und ihren Gemischen ausgewählt ist, wobei das Lösemittel in einer Menge unter 20 Gew.-%, bezogen auf das Gesamtgewicht der Zusammensetzung, enthalten ist und wobei das Gewichtsverhältnis des Dimethylethers zu dem oder den weiteren Treibmitteln im Bereich von 95/5 bis 50/50 liegt, wobei die Zusammensetzung 20 bis 90 Gew.-% Wasser enthält, bezogen auf das Gesamtgewicht der Zusammensetzung, wobei der Dimethylether in Mengen enthalten ist, die im Bereich von 4 bis 35 Gew.-% liegen, bezogen auf das Gesamtgewicht der Zusammensetzung.

2. Zusammensetzung nach Anspruch 1, **dadurch gekennzeichnet, dass** das anionische Polymer ausgewählt ist unter:
- den Polymeren, die Carbonsäure-Einheiten enthalten, die von ungesättigten Mono- oder Dicarbonsäure-Monomeren der Formel 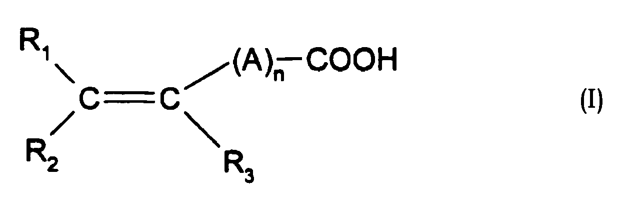 abgeleitet sind, worin bedeuten:
n eine ganze Zahl von 0 bis 10,
A eine Methylengruppe, die gegebenenfalls mit dem Kohlenstoffatom der ungesättigten Gruppe oder, wenn n größer als 1 ist, der benachbarten Methylengruppe über ein Heteroatom, wie Sauerstoff oder Schwefel, verbunden ist,
R₁ Wasserstoff, Phenyl oder Benzyl,
R₂ Wasserstoff, niederes Alkyl oder eine Carboxygruppe,
R₃ Wasserstoff, niederes Alkyl, eine Gruppe -CH₂-COOH, Phenyl oder Benzyl,
- den Polymeren, die von Sulfonsäure abgeleitete Einheiten enthalten, wie Vinylsulfon-, Styrolsulfon-, Ligninsulfon-, Acrylamidoalkylsulfon-Einheiten.

3. Zusammensetzung nach einem der vorhergehenden Ansprüche, **dadurch gekennzeichnet, dass** das anionische Polymer ausgewählt ist unter:
A) den Homo- oder Copolymeren von Acrylsäure oder Methacrylsäure und ihren Salzen, den Copolymeren aus Acrylsäure und Acrylamid und ihren Salzen, den Natriumsalzen von Polyhydroxycarbonsäuren,
B) den Copolymeren aus Acrylsäure oder Methacrylsäure und einem einfach ethylenisch ungesättigten Monomer, wie Ethylen, Styrol, Vinylester, Acrylsäure- oder Methacrylsäureester, die gegebenenfalls auf ein Polyalkylenglykol, wie Polyethylenglykol, gepfropft sind und gegebenenfalls vernetzt sind, den Copolymeren dieses Typs, die in ihrer Kette eine Acrylamid-Einheit enthalten, die gegebenenfalls N-alkyliert und/oder N-hydroxyalkyliert ist, den Copolymeren aus Acrylsäure und C₁-C₄-Alkylmethacrylat und den Terpolymeren aus Vinylpyrrolidon, Acrylsäure und C₁-C₂₀-Alkylmethacrylat,
C) den Copolymeren, die von Crotonsäure abgeleitet sind, wie den Copolymeren, die in ihrer Kette Vinylacetat- oder Vinylpropionat-Einheiten und gegebenenfalls weitere Monomere, wie Allylester oder Methallylester, Vinylether oder Vinylester einer gesättigten, geradkettigen oder verzweigten Carbonsäure mit langer Kohlenwasserstoffkette, wie der Carbonsäuren, die mindestens 5 Kohlenstoffatome aufweisen, enthalten, wobei diese Polymere gegebenenfalls gepfropft und vernetzt sein können,
D) den Polymeren, die abgeleitet sind von Maleinsäure, Fumarsäure, Itaconsäure und ihren Anhydriden mit Vinylestern, Vinylethern, Vinylhalogeniden, Phenylvinyl-Derivaten, Acrylsäure und ihren Estern, den Copolymeren aus Maleinsäure-, Citraconsäure-, Itaconsäureanhydrid und einem Allylester oder Methallylester, die gegebenenfalls eine Acrylamid-, Methacrylamid-Gruppe, ein α-Olefin, einen Acrylester oder Methacrylester, eine Acrylsäure oder Methacrylsäure oder Vinylpyrrolidon in ihrer Kette enthalten, wobei die Anhydridgruppen einfach verestert oder einfach amidiert sind,
E) den Polyacrylamiden, die Carboxylat-Gruppen enthalten.

4. Zusammensetzung nach einem der vorhergehenden Ansprüche, **dadurch gekennzeichnet, dass** das anionische Polymer ausgewählt ist unter:
- den Acrylsäure-Copolymeren, wie dem Acrylsäure/Ethylacrylat/ N-*tert*.-Butylacrylamid-Terpolymer,
- den von Crotonsäure abgeleiteten Copolymeren, wie den Vinylacetat/ tert.-Butylbenzoesäurevinylester/ Crotonsäure-Terpolymeren und den Crotonsäure/Vinylacetat/Neododecansäurevinylester-Terpolymeren,
- den Polymeren, die abgeleitet sind von Maleinsäure, Fumarsäure, Itaconsäure oder den entsprechenden Anhydriden mit Vinylestern, Vinylethern, Vinylhalogeniden, Phenylvinyl-Derivaten, Acrylsäure und ihren Estern, wie den Copolymeren aus dem Methylvinylether und einfach verestertem Maleinsäureanhydrid.

5. Zusammensetzung nach einem der Ansprüche 1 bis 4, **dadurch gekennzeichnet, dass** das schaumbildende kationische Polymer ausgewählt ist unter:
- den vernetzten Polyamin/Polyglykol-Copolymeren,
- den kationischen Celluloseethern der folgenden Formel (II) 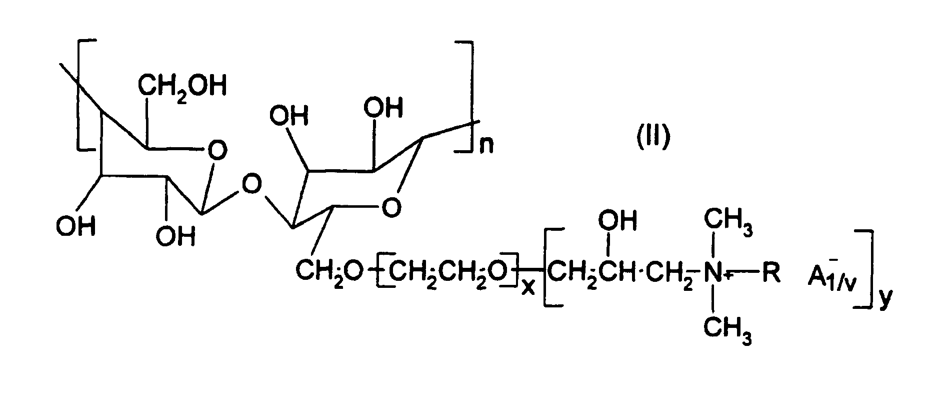 in der bedeuten:
R einen Alkylrest mit mindestens 8 Kohlenstoffatomen, vorzugsweise 10 bis 24 Kohlenstoffatomen und besser 10 bis 18 Kohlenstoffatomen,
A⁻ ein Anion oder ein Gemisch von Anionen,
v eine Zahl, die der Wertigkeit von A entspricht,
n eine ganze Zahl im Bereich von 50 bis 20000, vorzugsweise
100 bis 6000 und besser 250 bis 4000,
x eine ganze Zahl im Bereich von 0 bis 6 und
y eine ganze Zahl im Bereich von 0 bis 3, vorzugsweise 0 bis 2, mit der Maßgabe, dass der Grad der kationischen Substitution KS größer als 0 ist.

6. Zusammensetzung nach einem der vorhergehenden Ansprüche, **dadurch gekennzeichnet, dass** das oder die Polymere in einer Konzentration von 0,1 bis 15 Gew.-%, bezogen auf das Gesamtgewicht der Zusammensetzung, enthalten sind.

7. Zusammensetzung nach Anspruch 6, **dadurch gekennzeichnet, dass** das oder die Polymere in einer Konzentration von 0,5 bis 10 Gew.-%, bezogen auf das Gesamtgewicht der Zusammensetzung, enthalten sind.

8. Zusammensetzung nach einem der vorhergehenden Ansprüche, **dadurch gekennzeichnet, dass** das Alkan unter Isobutan, Propan, Butan, Pentan, Isopentan und den Gemischen dieser Alkane ausgewählt ist.

9. Zusammensetzung nach einem der Ansprüche 1 bis 7, **dadurch gekennzeichnet, dass** das Halogenalkan unter den Difluorethanen, den Tetrafluorethanen, Octafluorcyclobutan und den Gemischen dieser Halogenalkane ausgewählt ist.

10. Zusammensetzung nach einem der Ansprüche 1 bis 8, **dadurch gekennzeichnet, dass** das Treibmittelgemisch aus Dimethylether und Isobutan besteht.

11. Zusammensetzung nach einem der vorhergehenden Ansprüche, **dadurch gekennzeichnet, dass** das Gemisch aus Dimethylether und dem oder den weiteren Treibmittel(n) in einer Menge von 5 bis 55 Gew.-%, bezogen auf das Gesamtgewicht der Zusammensetzung, enthalten ist.

12. Zusammensetzung nach Anspruch 11, **dadurch gekennzeichnet, dass** das Gemisch aus Dimethylether und dem oder den weiteren Treibmittel(n) in einer Menge von 15 bis 35 Gew.-%, bezogen auf das Gesamtgewicht der Zusammensetzung, enthalten ist.

13. Zusammensetzung nach einem der vorhergehenden Ansprüche, **dadurch gekennzeichnet, dass** das oder die weiteren Treibmittel in einer Menge von 1 bis 20 Gew.-%, bezogen auf das Gesamtgewicht der Zusammensetzung, enthalten sind.

14. Zusammensetzung nach einem der vorhergehenden Ansprüche, **dadurch gekennzeichnet, dass** das Gewichtsverhältnis des Dimethylethers zu dem oder den Treibmittel(n) im Bereich von 90/10 bis 55/45 liegt.

15. Zusammensetzung nach einem der vorhergehenden Ansprüche, **dadurch gekennzeichnet, dass** sie ein anionisches Polymer mit Schaumvermögen in Kombination mit einem kationischen Polymer, das schaumbildend oder nicht schaumbildend wirkt, enthält.

16. Zusammensetzung nach einem der vorhergehenden Ansprüche, **dadurch gekennzeichnet**, sie ein Copolymer aus Methylvinylether und einfach verestertem Maleinsäureanhydrid und ein Hydroxyethylcellulose/Diallyldimethylammoniumchlorid-Copolymer enthält.

17. Aerosolbehälter, **dadurch gekennzeichnet, dass** er eine wie in einem der vorhergehenden Ansprüchen definierte Zusammensetzung enthält.

18. Verfahren zur Behandlung von Keratinmaterialien, **dadurch gekennzeichnet, dass** auf diese Materialien mindestens eine Zusammensetzung, die wie in einem der Ansprüche 1 bis 16 definiert ist, aufgetragen wird.

19. Verwendung einer Zusammensetzung nach einem der Ansprüche 1 bis 16 als Zusammensetzung, die vor oder nach einer beliebigen Haarbehandlung, wie einer Haarwäsche, Färbung oder Entfärbung, einer Dauerwellbehandlung oder Entkräuselung, aufgetragen wird, als Zusammensetzung zum Legen einer Wasserwelle, zur Frisurengestaltung mit einer Fönbürste ("brushing"), zur Frisurengestaltung, als Frisierzusammensetzung, als Zusammensetzung zur Färbung, Entfärbung, Dauerwellbehandlung oder zur Entkräuselung der Haare.
